# EUROPEAN PATENT APPLICATION

(11) **EP 2 147 666 A1**
(43) Date of publication of application: **27.01.2010**
(21) Application number: 09386019.5
(22) Date of filing: 27.07.2009
(51) Int. Cl.: A61F 15/00

(54) **Riddled with holes sleeves for the glans penis' protection**

(30) Priority: 25.07.2008 GR 20080100495
(71) Applicant: Nikolopoulos, P. Vasilios, 19400 Koropi Attikis (GR)
(72) Inventor: Nikolopoulos, P. Vasilios, 19400 Koropi Attikis (GR)

(57) **Abstract**

The riddled with holes sleeve for the glans penis' protection is made up by an hypoallergenic material such as riddled with holes papercoton, gauze, elastic textile or other similar material that has openings all over its surface.

The advantage of this invention is that it manages not only to keep the cleanness of the area of the glans penis all day long but also to move away the hairs which worms it's way and provokes itch and irritation.

The riddled with holes sleeve for the glans penis' protection for both shapes, is produced under pressure and temperature in moulds inox.

## Description

### Description of the invention:

The invention relates to a riddled with holes sleeve for the glans penis' protection of the male organ from microorganisms which brings not only bad smell in the area of the glans penis but also excitement in the skin which encloses the prepuse.

In the Greek and international market today, it doesn't seem that exists similar or same design for the protection of the part of the human being.

From the nature the male organ is covered from the foreskin that encloses the glans penis and as a result there isn't sufficient airing so it causes excitement which is usually a consequence of the insufficient cleanness of the penis and the development of bacterias and fungi which grow in areas where there is damp.

The advantage of this invention is that it manages not only to keep the cleanness of the area of the glans penis all day long but also to move away the hairs which worms it's way and provokes itch and irritation.

The invention bases, as it concerns the shape, in the well known male condom. The riddled with holes sleeve for the glans penis' protection can be produced by a widely range of hypoallergenic materials such as riddled with holes papercoton, gauze, special elastic textile which has openings.Whichever material will be used for his construction it will have openings all over the surface.

The construction of the riddled with holes sleeve for the glans penis' protection becomes in prefabricated moulds inox where under the appropriate pressure and temperature solidifies and it comes out ready after the disconnection of the moulds.
The riddled with holes sleeve for the glans penis' protection will have a small cutting in the front part so as to let free passage for urination and it fits in the base of penis with a very smooth elastic ring.As an alternative it can be produced another model which has a different base of supporting as it leads to the testicles from where it can be hold up during walking and offering an excellent comfort to the user.

The invention described is unique and original full of beneficient resultats for the human being.

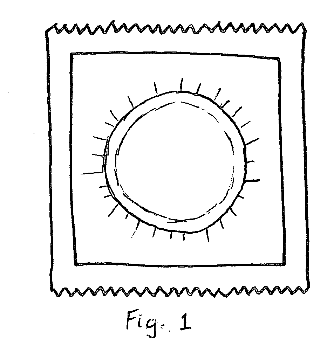

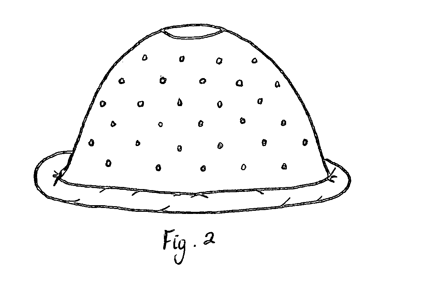

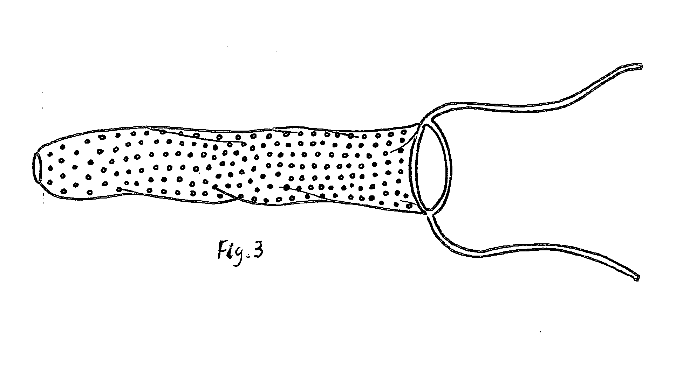

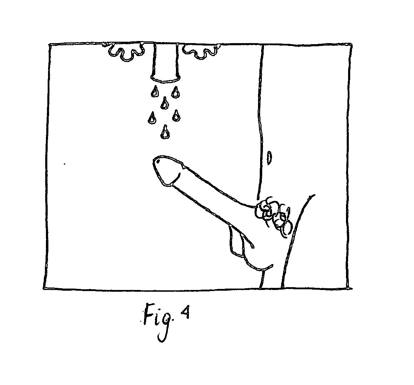

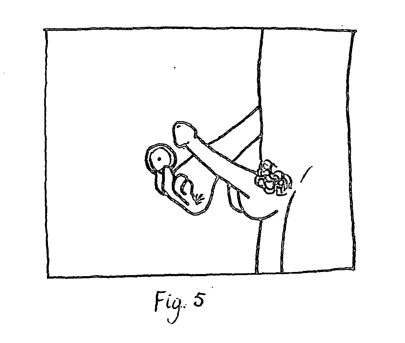

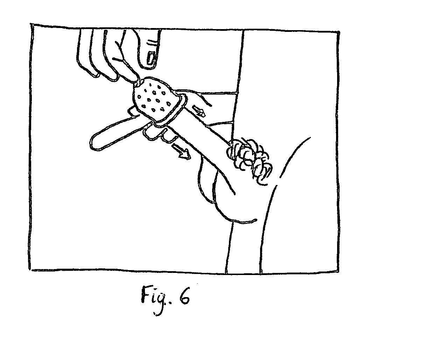

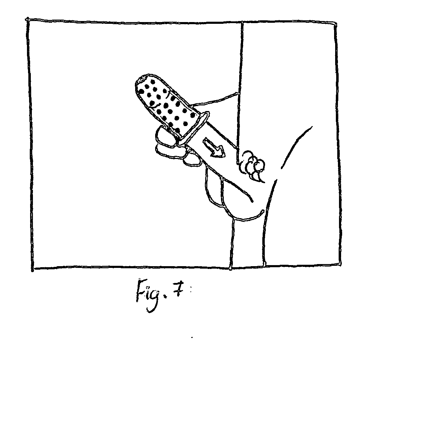

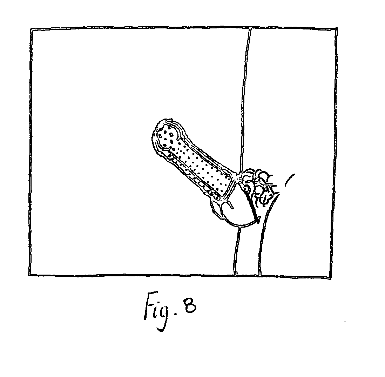

## Claims

1. Riddled with holes sleeve for the glans penis'protection is **characterized** because of the shape of the male condom and it has not only openings all over its length and surface but also in the front part so as to let free passage for urination and this sleeve fits in the base of the male organ with a very smooth elastic ring for his holdingup.

2. Riddled with holes sleeve for the glans penis' protection according to the demande 1 is **characterized** because the base of supporting leads to the testicles from where it can be hold up.

3. Riddled with holes sleeve for the glans penis'protection according to the demand 1 is **characterized** because it's produced in prefabricated moulds inox where under the appropriate pressure and temperature solidifies and it comes out ready after the disconnection of the moulds.

4. Riddled with holes sleeve for the glans penis' protection according to the demands 1-3 is **characterized** because it's produced by hypoallergenic materials such as papercoton, gauze elastic or a combination by them.

5. Use of the riddled with holes sleeve for the glans penis' protection from microorganisms and excitements in the skin.
